# EUROPEAN PATENT SPECIFICATION

(11) **EP 3 092 962 B1**
(45) Date of publication and mention of the grant of the patent: **04.10.2017**
(21) Application number: 15167425.6
(22) Date of filing: 12.05.2015
(51) Int. Cl.: A61B 17/70

(54) **INSTRUMENT FOR USE WITH A POLYAXIAL BONE ANCHORING DEVICE AND SYSTEM INCLUDING THE INSTRUMENT AND A POLYAXIAL BONE ANCHORING DEVICE**
INSTRUMENT ZUR VERWENDUNG MIT EINER POLYAXIALEN KNOCHENVERANKERUNGSVORRICHTUNG UND SYSTEM MIT DEM INSTRUMENT UND POLYAXIALE KNOCHENVERANKERUNGSVORRICHTUNG
INSTRUMENT DESTINÉ À ÊTRE UTILISÉ AVEC UN DISPOSITIF D'ANCRAGE OSSEUX POLYAXIAL ET SYSTÈME COMPRENANT LEDIT INSTRUMENT ET UN TEL DISPOSITIF D'ANCRAGE

(43) Date of publication of application: 16.11.2016
(73) Proprietor: Biedermann Technologies GmbH & Co. KG, 78166 Donaueschingen (DE)
(72) Inventor: Biedermann, Lutz, 78048 VS-Villingen (DE)
(74) Representative: Prüfer & Partner mbB Patentanwälte · Rechtsanwälte

(56) References cited:
- US-A1- 2004 147 937
- US-A1- 2006 036 244
- US-A1- 2014 188 173

## Description

The invention relates to an instrument for use with a polyaxial bone anchoring device and to a system including the instrument and a polyaxial bone anchoring device. Such a polyaxial bone anchoring device includes a receiving part for receiving a rod and for accommodating a head of a bone anchoring element. The head can be clamped by exerting pressure onto it via a clamping element. The instrument comprises a first member and a second member displaceable relative to the first member wherein the first member is configured to engage the receiving part in a positive-fit manner and the second member is configured to engage the clamping element for adjusting clamping force onto the head.

A variety of tools for use with polyaxial bone anchors are known. For example US 2011/0004222 A1 describes a tool comprising a tubular counter-holding portion that is configured to engage a receiving part of a bone anchor and a driven shaft extending through the counter-holding portion, wherein the driven shaft is configured to engage a locking element of the bone anchor, for example a set screw. With the driven shaft the locking element is tightened while the receiving part is held with the counter-holding portion.

Further, US 2004/0147937 A1 discloses a spinal rod approximator for seating a stabilizing rod in a rod-receiving portion of a spinal implant. To this end, the spinal rod approximator includes a body having at its distal end two gripping branches for gripping a spinal implant head and, between the branches, a substantially U-shaped recess for accommodating a spinal rod. For inserting a closure mechanism for the rod, an inserter shaft is slidably and rotatably received within an interior channel of the body.

US 8,926,671 describes a receiving part for receiving a rod for coupling the rod to a bone anchoring element wherein the receiving part comprises a receiving part body for accommodating a head of a bone anchoring element and a pressure element with a flexible portion to clamp an inserted head. The pressure element is movable along a longitudinal axis of the receiving part body from an insertion position to insert the head and to a pre-locking position wherein the head is clamped in the receiving part by a pre-stress exerted by the pressure element, to a locking position to lock the head in the receiving part. The pre-stress exerted by the pressure element allows to maintain a desired angular position of the bone anchoring element relative to the receiving part by friction before the head of the bone anchoring element is finally locked.

In the case of an in-situ placement of the receiving part onto a bone anchoring element that has been already inserted into a bone or a vertebra it is advantageous when the mounting of the receiving part with the pressure element onto the head of the bone anchor can be performed with low force. However, if the insertion force is low, the pre-stress exerted onto the head of the bone anchoring element might be too low for the further procedure of aligning the receiving part for inserting the rod.

It is the object of the invention to provide an instrument for use with a polyaxial bone anchoring device and a system of an instrument and an improved polyaxial bone anchoring device that facilitates the handling during surgery.

The object is solved by an instrument according to claim 1 and by a system of an instrument and a polyaxial bone anchoring device according to claim 11 or 12. Further developments are given in the dependent claims.

The instrument allows to adjust the friction force that is exerted onto the head of the bone anchoring element in an easy manner during surgery. In particular, the instrument in connection with a polyaxial bone anchoring device allows to adjust the friction force onto the head manually by actuating the second member of the instrument with one hand while holding the receiving part with the first member held by the other hand. Pivoting the receiving part relative to the bone anchor with the first member gives the surgeon an immediate feedback of the friction force acting onto the head. This permits a precise adjustment of the desired friction force based onto the sensing of the friction force with the surgeon's hands.

The instrument may be particularly useful for a polyaxial bone anchoring device of the bottom loading type, wherein the head of the bone anchoring element is inserted from a bottom end into the receiving part. The receiving part can be placed in-situ onto the head of the bone anchoring element wherein the shank has already been inserted into the bone. Hence, an instrument for use with an in-situ type bottom loading bone anchoring device is provided wherein the friction force acting onto the head can be adjusted in-situ on a patient.

The first member of the instrument may be connected to the receiving part in the positive-fit manner wherein a surface of the receiving part cooperates with a surface of the first member to prevent rotation between the first member and the receiving part.

The second member connectable to the first member only in a specific position relative to the first member. In this position an engagement portion of the second member is substantially aligned with an engagement portion of a clamping element of the polyaxial bone anchoring device such that the clamping element can be easily engaged. Therefore, the procedure of finding the clamping element with the instrument is facilitated and the actuation of the clamping element can be performed quickly. With the clamping element the friction force onto the head of the anchoring element can be increased by rotating the clamping element in one direction and can be decreased again by rotating it in the opposite direction. By the manual adjustment of the frictional clamping of the head before final locking the procedure of aligning a plurality of receiving parts for inserting the rod is considerably simplified.

Further features and advantages will become apparent from the description of embodiments by means of the accompanying drawings. In the drawings:
- Fig. 1: shows a perspective view of an embodiment of the instrument together with a portion of the spinal column and an inserted polyaxial bone anchoring device;
- Fig. 2a: shows a perspective exploded view of the instrument of Fig. 1;
- Fig. 2b: shows an enlarged view of a detail of Fig. 2a;
- Fig. 3: shows a perspective exploded view of the polyaxial bone anchoring device according to an embodiment;
- Fig. 4: shows a cross-sectional view of the polyaxial bone anchoring device of Fig. 3 wherein the cross-section is taken in a plane perpendicular to the rod axis;
- Fig. 5: shows a perspective view from above of an outer tubular member of the instrument seen from above;
- Fig. 6: shows a perspective view from the bottom of the outer tubular member shown in Fig. 5;
- Fig. 7: shows a top view of the outer tubular member of Figs 5 and 6;
- Fig. 8a: shows a cross-sectional view of the outer tubular member of Figs. 5 and 6 along line A - A in Fig 7;
- Fig. 8b: shows a cross-sectional view of an enlarged portion of the front end of the outer tubular member shown in Fig. 8a;
- Fig. 9: shows a perspective view from above of an inner member of the instrument;
- Fig. 10a: shows a perspective view from the bottom of the inner member of Fig. 9;
- Fig. 10b: shows a perspective view of an enlarged portion of a front end of the inner member of Figs. 9 and 10 a;
- Figs. 11aa to 11c: show cross-sectional views of steps of mounting the instrument to a polyaxial bone anchoring device with the bone anchoring element already inserted into a bone, the cross-section taken in a plane containing the rod axis;
- Figs. 12a to 12c: show partial cross-sectional views of steps of mounting the instrument to a polyaxial bone anchoring device.

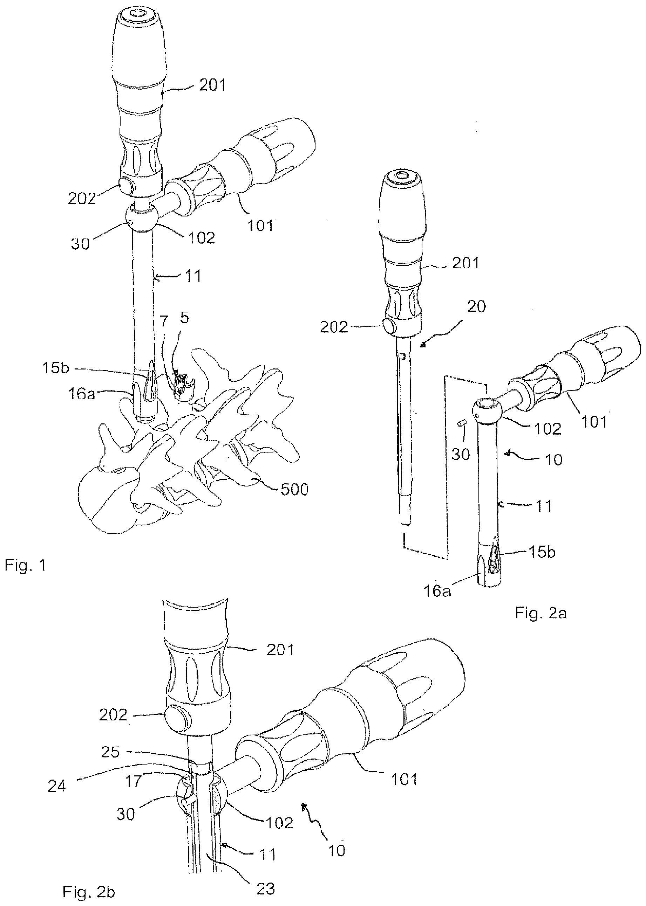

Referring to Figs. 1 to 2b, and instrument according to an embodiment includes a first member 10 and a second member 20 that is insertable into the first member 10. The first member 10 is configured to engage a receiving part 5 of a polyaxial bone anchoring device. In Fig. 1, the receiving part 5 is pivotably connected to a bone anchoring element that has been already inserted into the pedicle of a vertebra 500. A portion of the second member 20 is configured to engage a clamping element 7 provided in the receiving part 5 and to rotate the clamping element 7.

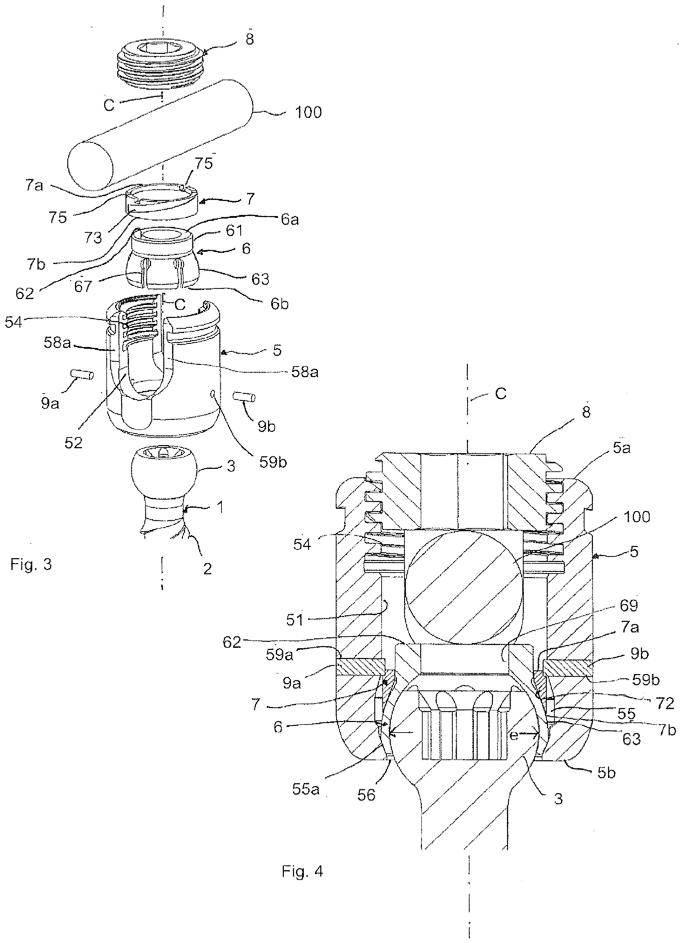

Next an embodiment of the polyaxial bone anchoring device will be described with reference to Figs. 3 and 4. The polyaxial bone anchoring device includes a bone anchoring element 1, for example in the form of a bone screw having a threaded shaft 2 and a head 3 that may be a spherical segment-shaped head. The bone anchoring device further comprises the receiving part 5 for receiving a rod 100 to connect the rod 100 to the bone anchoring element 1. Further, a pressure element 6 is provided for exerting pressure onto the inserted head 3 of the bone anchoring element to clamp and finally lock the head 3 in the receiving part 5. Additionally, the clamping element 7 is arranged in the receiving part 5 around a portion of the pressure element 6 wherein the clamping element is adapted for exerting a compression force onto the pressure element 6 to increase the pressure force onto an inserted head 3. The bone anchoring device further includes a locking element 8 for securing the rod 100 in the receiving part 5 and for exerting a force to lock the head 3 in the receiving part 5. The locking element 8 may be, for example, a set screw.

The receiving part 5 comprises a first end 5a and a second end 5b opposite the first end and an axis of symmetry C passing through the first end 5a and the second end 5b. A passage 51 is provided that extends from the first end 5a to the second end 5b and that is substantially rotationally symmetric to the axis of symmetry C. In a first region adjacent to the first end 5a, the receiving part 5 comprises a substantially U-shaped recess 52 that is symmetric with respect to the axis C, the recess 52 having a bottom directed towards the second end 5b. An internal thread 54 is formed adjacent or near the first end 5a that cooperates with the locking element 8. The channel formed by the substantially U-shaped recess 52 is sized so as to receive the rod 100 therein, wherein the rod is configured to connect a plurality of anchoring devices.

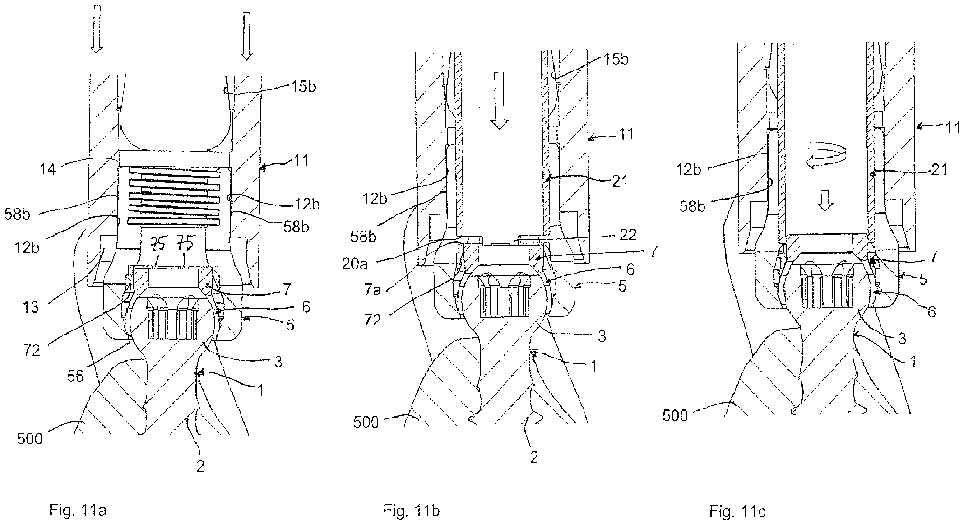

As can be seen in particular in Fig. 4, in a lower portion of the receiving part, an accommodation space 55 that has an inner diameter greater than an inner diameter of the passage 51 in the upper section of the receiving part 5 is provided. The accommodation space 55 narrows towards the second end 5b in a narrowing portion 55a. The size of the accommodation space 55 is such that the head 3 of the bone anchoring element 1 and a lower portion of the pressure element 6 can be accommodated therein. The accommodation space has an opening 56 at the bottom end 5b of the receiving part. A diameter of the opening 56 is greater than a greatest diameter of the head 3 so that the head 3 is insertable through the opening 56 at the bottom end 5b into the receiving part 5. Flat outer surface portion 58a, 58b on the receiving part 5 on both sides of the U-shaped recess 52 (Figs. 3, Fig. 11a) are configured to cooperate with flat surfaces at the instrument to provide a positive-fit connection between the receiving part 5 and the instrument. Moreover, on each side of the channel, at a position of approximately 90° with respect to the channel axis, bores 59a, 59b extending through the wall of the receiving part 5, respectively, are provided for receiving pins 9a, 9b.

Referring further to Figs. 3 and 4, the pressure element 6 has a first end 6a and an opposite second end 6b. Adjacent to the first end 6a the pressure element has a first portion 61 that is substantially cylindrical and that has an outer diameter that is smaller than an inner diameter of the passage 51. At the first end 6a a rod support surface 62 is provided.

Between the cylindrical first portion 61 and the second end 6b there is a second cap-like portion 63 with a hollow interior having a shape that is adapted to receive the head 3 therein. In particular, the hollow interior has a spherical shape with such a length in axial direction that it can accommodate the head 3 with its greatest diameter e (see Fig.4) therein. The second portion 63 of the pressure element 6 further comprises at least one vertical slit 67, preferably a plurality of slits 67 that are open to the second end 6b and extend through the second portion 63 almost up to the first portion 61 to render the second portion flexible. Further, the pressure element 6 comprises a coaxial bore 69 for providing access to the screw head 3 by a screw-driver. The second portion 63 of the pressure element 6 is adapted to exert a pressure onto an inserted head 3 and to hold the head 3 by a frictional force between the inner surface of the hollow interior and the outer surface of the head 3. In other words, the flexible portion 63 fits tightly onto the head 3. Because the second portion 63 has a flexible wall, the second portion 63 can expand within the accommodation space 55 when the screw head 3 is inserted. When the pressure element 6 is pressed downward such that it enters the narrowing portion 55a of the receiving part 5, the head 3 cannot be removed through the lower opening 60 and the head is maintained at an angular position with respect to the receiving part 5 by the pre-stress of the pressure element 6 that acts onto the head 3.

The clamping element 7 is a ring-shaped part with a first end 7a and an opposite second end 7b and a substantially cylindrical outer shape. As depicted in Fig. 4 the clamping element 7 comprises an internal surface 72, which widens towards the second end 7b and which may be conical. The size of the internal surface 72 is such that when the clamping element 7 is mounted, the surface 72 encompasses an upper region of the flexible second portion 63 of the pressure element 6. By a downward movement of the clamping element 7 the internal surface 72 contacts the outer surface of the flexible portion 63 so that pressure is exerted onto the flexible portion resulting in an increased compression of the head 3. Thereby, the frictional force between the head 3 and the pressure element6 is increased.

An advancement structure including two opposite helical grooves 73 one of which is seen in Fig. 3 are provided on the outer surface of the clamping element 7. The helical grooves 73 are open towards the first end 7a and have a closed end towards the second end 7b. The size is such that the pins 9a, 9b can engage the grooves. The grooves 73 and the pins 9a, 9b permit to advance the clamping element 7 in the receiving part 5 through rotating the clamping element 7 around the axis of symmetry C. When the pressure element 6 and the clamping element 7 are inserted into the receiving part 5, the closed ends of the grooves 73 prevent the clamping element 7 to escape through the top end 5a. The advancement structure permits a stepless advancement of the clamping element 7 along the central axis C. A size of the helical groove 73 and the pins 9a, 9b and/or a pitch of the groove may be selected such that the clamping element stays in a position if no torque is applied.

As further depicted in Figs. 3 and 4, the clamping element 7 comprises in the surface of the first end 7a a plurality of engagement recesses 75 that are configured to be engaged with the instrument so that torque can be applied with the instrument onto the clamping element 7. More specifically, in the embodiment, the engagement recesses are arranged in pairs on opposite sides of the central axis C and preferably 90° offset from the upper open upper end of the grooves 73.

The parts of the bone anchoring device can be made of a bio-compatible material, such as a bio-compatible metal or a bio-compatible metal alloy, for example stainless steel, titanium, NiTialloys, such as Nitinol, magnesium or magnesium alloys or from a bio-compatible plastic material, such as, for example, polyether ether ketone (PEEK) or poly-1-lactide acid (PLLA). The parts can be made of the same or of different materials.

The instrument will be explained more in detail referring first to Figs. 1 to 3 and 5 to 8b. The first member 10 comprises a tubular portion 11 forming an outer tubular member of the instrument. The tubular portion 11 comprises a front end 11a and an opposite rear end 11b. Near the front end 11a of the tubular two opposite flat surfaces 12a, 12b are provided that are configured to cooperate with the corresponding flat surfaces 58a, 58b at the receiving part 5. The tubular portion 11 further has a portion 13 near the front end 11a with a greater inner diameter than the distance between the flat surfaces 12a, 12b. The portion 13 may widen towards the front end 11a in order to facilitate placement of the tubular portion 11 over the receiving part 5. At a distance from the front end 11a a protrusion 14 may be formed that may extend circumferentially at the inner wall of the tubular portion 11 and that serves as a stop for the first end 5a of the receiving part 5. It shall be noted that the protrusion 14 may form a full annular protrusion or max also extend only along a portion of the inner circumference. The distance of the protrusion 14 from the front end 11a is such that when the tubular portion 11 is placed onto the receiving part 5 and the first end 5a of the receiving part 5 abuts against the protrusion 14, the flat surfaces 12a, 12b can engage the corresponding flat surfaces 58a, 58b at the receiving part.

At substantially 90° offset from the flat surface portions 12a, 12b, two triangular windows 15a, 15b are formed in the wall of the tubular portion 11. The orientation of the triangular windows 15a, 15b is such that the greatest diameter is facing towards the front end 11a. The windows 15a, 15b allow inspection of the placement of the instrument and may facilitate cleaning of the instrument. A wall thickness of the tubular portion 11 in the region adjacent to the front end 11a mostly up to the end of the windows 15a, 15b may be increased to increase the strength of the portion of the instrument that engages the receiving part 5. At a position corresponding to the position of the flat surfaces 12a, 12b, substantially flat surface portions 16a, 16b are formed at the outer wall of the tubular portion 11. These flat surface portions 16a, 16b result in a reduced outer diameter of the tubular portion in the direction perpendicular to the rod axis. This allows to use the instrument even in cases where the receiving parts of adjacent bone anchors are positioned very close together.

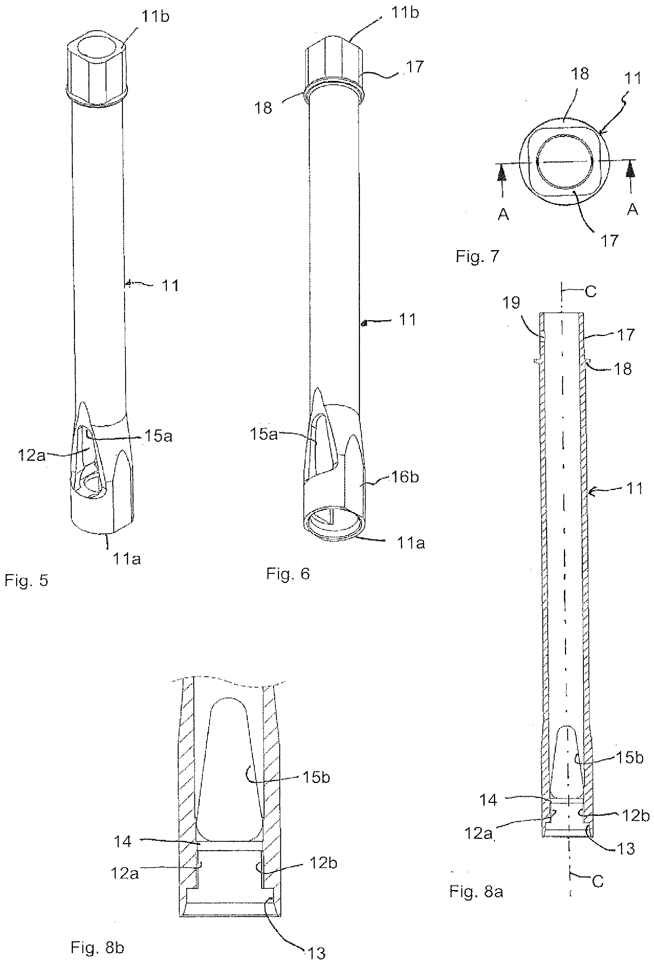

Adjacent to the rear end 11b, an outer contour of a portion 17 of the tubular portion may be square-shaped. At the end of the square-shaped portion 17 facing away from the rear end 11b, a stop 18 is provided for limiting the axial position of a handle portion on the tubular portion 11 as described below. The stop 18 may be an annular projection. At a distance from the rear end 11b a transverse hole 19 is provided that extends through the wall of the square-shaped portion 17 for receiving a pin 30. The pin 30 has a length such that it can protrude into the interior of the tubular portion 11. The orientation of the transverse hole 19 is such that the hole 19 is in a circumferential direction substantially aligned with the center of the flat surface portions 12a on one side (Fig. 8a).

Turning again to Figs. 1 to 2b, a handle portion 101 is mounted to the tubular portion 11. The handle portion 101 comprises a mounting portion 102 that is tubular and has an inner contour corresponding to the outer contour of the square-shaped portion 17, so that the handle portion 101 is connected via a positive-fit connection to the tubular portion 11. The mounting portion 102 is prevented from moving towards the front end 11a by the stop 18. The handle portion 101 extends substantially perpendicular to the tubular portion 11. A transverse hole 103 is provided in the mounting portion 102 at a position offset by 180° from the handle portion 101. In the mounted state, the hole 103 in the mounting portion 102 and the hole 19 in the tubular portion 11 are aligned, so that the pin 30 can extend through both holes.

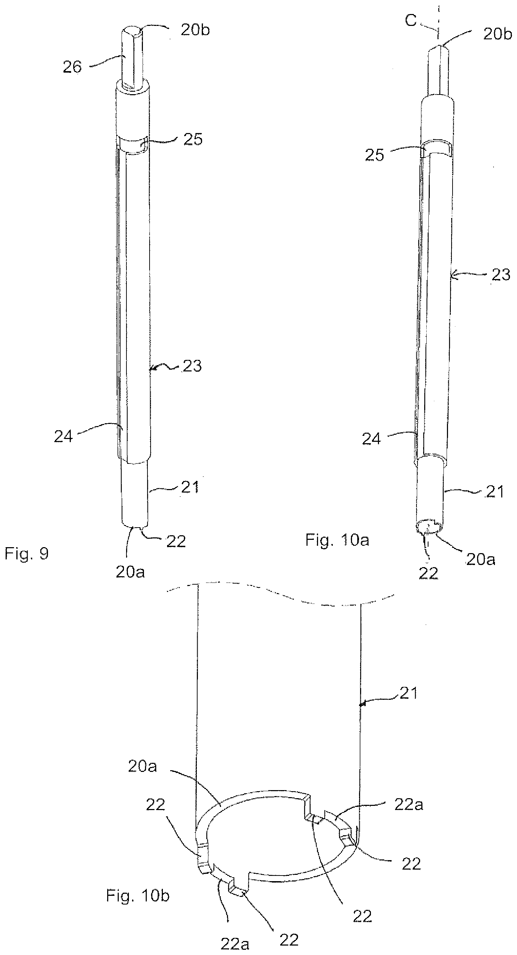

The second member 20 is depicted more in detail in Figs. 9 to 10b. It comprises a front end 20a and a rear end 20b. A portion 21 adjacent the front end 20a is tubular. The tubular portion 21 has an outer diameter that is smaller than an inner diameter of the passage 51 in the receiving part 5 such that the tubular portion 21 can be inserted into the receiving part 5 from the first end 5a. More in detail an outer diameter of the tubular portion 21 may be substantially the same as an outer diameter of the clamping element 7 at the first end 7a which can be seen for example in Fig. 11b. An inner diameter of the tubular portion 21 is greater than an outer diameter of the first portion 61 of the pressure element 6 so that the tubular portion 21 can slide over the first portion 61 of the pressure element as depicted in Fig. 11c. A plurality of engagement projections 22 are provided at the front end 20a The engagement projections 22 are sized and shaped to engage the recesses 75 provided at the first end 7a of the clamping element 7. In the example shown four engagement recesses 22 are arranged in two pairs that are located on opposite sides from the central axis C. The circumferential distance between two engagement recesses 22 of one pair is smaller then the distance between the pairs. A wall portion 22 a between the projections 22 of one pair of projections projects farther than the front end 20a of the tubular portion 21. Hence, when the projections 22 engage the recesses 75 of the clamping element 7, a gap is between the first end 7a and the front end 20a of the tubular portion 21 (Fig. 11b). Thereby, a frictional contact between the tubular portion 21 and the clamping element 7 may be avoided.

The second member 20 comprises adjacent to the tubular portion 21 a cylindrical portion 23. The cylindrical portion 23 can be solid, i.e. needs not to be tubular. The cylindrical portion 23 has an outer diameter that is greater than the outer diameter of the tubular portion 21 but smaller than an inner diameter of the tubular portion 11 of the first member 10 such that the cylindrical portion 23 can slide with in the tubular portion 11 of the first member 10. A longitudinal groove 24 extends from the end of the cylindrical portion 23 that is adjacent to the tubular portion 21 to a distance from the opposite end of the cylindrical portion 23 substantially parallel to the central axis C. The width of the groove 24 is such that a front end of the pin 30 can be guided therein. In a circumferential direction the groove 24 is located substantially in the middle between the two pairs of projections 22. At the end of the longitudinal groove 24 facing the rear end 20b a horizontal recess 25 is provided into which the longitudinal groove 24 opens. The horizontal recess 25 is broader in the axial direction than the width of longitudinal groove 24. In particular the width of the horizontal recess 25 in an axial direction is greater than a diameter of the pin 30 so that the pin 30 can move slightly in the horizontal recess 25 in the axial direction. The horizontal recess 25 extends in a circumferential direction along approximately one quarter up to less then half of the circumference of the cylindrical portion 23. In a side view the longitudinal groove 24 and the horizontal recess 25 together have a substantially inverted L shape.

The longitudinal groove 24 forms a guiding structure that is configured to guide the insertion of the second member 20 into the tubular portion 11 of the first member 10 such that the position of the projections 22 at the front end 20a is aligned with the position of the recesses 75 of the clamping element 7. The horizontal recess 25 permits rotation of the second member 20 relative to the first member 10 limited by the abutment of the pin 30 against the horizontal ends of the horizontal recess 25. The width of the recess 25 in the axial direction allows the pin to move slightly in the recess 25 in the axial direction for finding the recesses 75 at the clamping element 7 and inserting the projections 22 therein.

At the end of the cylindrical portion 23 that is opposite to the tubular portion 21 a post 26 is provided that serves for a positive-fit connection with a handle 201. The handle 201 comprises a knob 202 that indicates the position of the longitudinal groove 24 in the circumferential direction. Additionally, the knob 202 may internally fix the post 26 in the handle portion 201. The instrument is also made of a body compatible material. Examples of such materials may be the same as described for the bone anchoring device.

The instrument is assembled as follows. The handle portion 101 of the first member is mounted onto the square-shaped portion 17 of the first member such that the holes 19, 103 overlap. Next the second member 20 is inserted into the tubular portion 11 of the first member 10 such that the longitudinal groove 24 is aligned with the holes 19, 103. Finally, the pin 30 is inserted into the holes 19, 103 until it protrudes into the longitudinal groove 24 as depicted in Fig. 2b. The knob 202 indicates the position of the longitudinal groove 24.

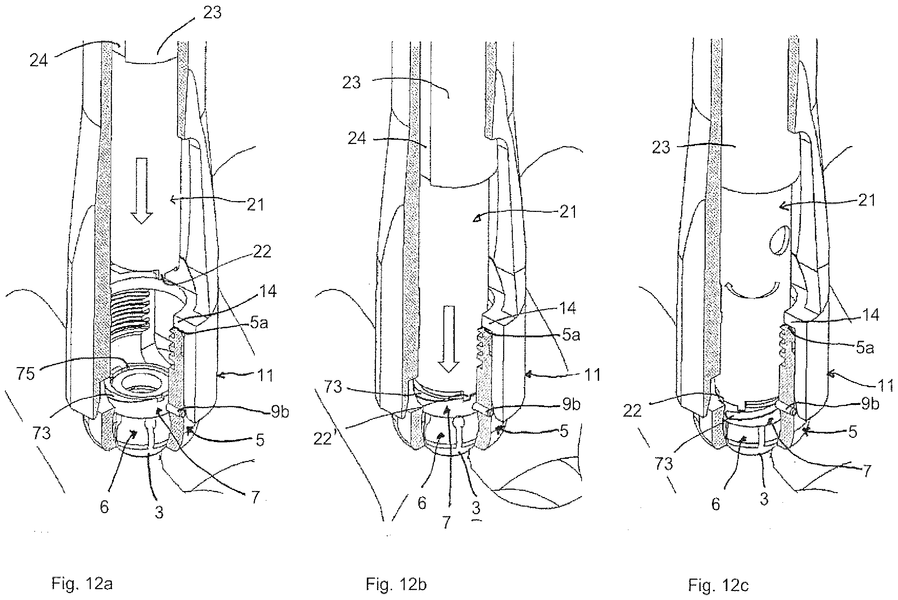

The use of the instrument will be explained referring to Figs. 11a to 11c and Figs. 12a to 12c. In a first alternative, the bone anchoring device is assembled in-situ. The bone anchoring element is already inserted into the bone and the receiving part 5 with the pressure element 6 and the clamping element 7 is mounted onto the head 3 thereafter. In a second alternative, the bone anchoring device is assembled outside the human body and the head 3 is inserted manually into the receiving part 5 through the lower opening 56.

The tubular portion 11 of the first member 10 is placed onto the receiving part 5 as depicted in Figs. 11a and 12a in such a manner that the inner flat surface portions 12a, 12b engage the flat surface portions 58a, 58b at the outside of the U- shaped recess 52. The first end 5a of the receiving part 5 abuts against the stop 14 in the tubular portion 11. By means of this the receiving part is firmly held by the first member 10. The clamping element 7 is arranged around the first portion 61 of the pressure element 6 in such a manner that the surface 72 does not press onto the flexible portion 63 of the pressure element 6. The recesses 75 are positioned at positions perpendicular to the rod axis.

Then, the second member 20 is inserted in the position in which the pin 30 can engage the longitudinal groove 24. The tubular portion 21 of the second member 20 is moved downward (arrow in Fig. 11a). Thereafter, as depicted in Figs. 11b and 12b the second member 20 is moved further downward so that the tubular portion 21 can enter into the receiving part 5. Thereby, the second member 20 is guided in an axial direction by the cooperation of the pin 30 with the longitudinal groove 24. The projections 22 are aligned with the recesses 75. The second member 20 is advanced until the projections 22 enter the recesses 75. In this relative axial position of the second member 20 relative to the first member 10 the pin 30 has entered the horizontal recess 25. Finally, as illustrated in Figs. 11c and 12c, the second member 20 is rotated relative to the first member 10 to apply torque to the clamping element 7. The helical grooves 73 of the clamping element 7 provide guidance for the pins 9a, 9b, so that clamping element 7 is advanced downward. While the downward advancement of the clamping element 7 the surface 72 of the clamping element contacts the outer surface of the flexible portion 63 of the pressure element 6 and compresses the flexible portion 63. Thereby, the pressure exerted onto the head 3 increases. An over advancement of the clamping element is prevented by the stop provided for the pin 30 at the end of the horizontal recess 25. To decrease the pressure, the second member 20 can be rotated in the opposite direction. The first member 10 acts as a counter holding member during the procedure.

By means of the above procedure, the frictional fit of the head 3 in the receiving part 5 can be easily and safely increased by rotating the second member relative to the first member. After a suitable friction force has been adjusted, first the second member 20 is retracted and then the first member 10 is decoupled from the receiving part 5. The receiving parts can be aligned whereby an angular position of the receiving part relative to the bone anchoring element is maintained due to the friction fit. Thereafter the rod is inserted and the whole construct is fixed by inserting and tightening the locking elements.

Modifications of the above described embodiments are conceivable. The cooperation surfaces on the first engagement member and the receiving part that prevent rotation of the first member relative to the receiving part may be shaped and arranged in an other manner to achieve a positive-fit connection. Only one single surface on the receiving part and one single surface at the first member may be sufficient. The engagement portions of the second member and the clamping element may have another shape and/or another arrangement. For example only one projection and a corresponding recess may be sufficient. The second member may also be placed around the first member and engage the clamping element from the outside of the receiving part. The longitudinal groove and the horizontal recess may be provided at the first member and a corresponding protrusion at the second member.

With regard to the polyaxial bone anchoring device, another clamping mechanism can be provided. For example, the clamping element can be advanced by a threaded connection between the receiving part and the clamping element. While a bottom loading polyaxial bone anchoring device has been shown, the instrument can also be used with a top loading polyaxial bone anchoring device in which the anchoring element is inserted into the receiving part from the first end. The head of the bone anchoring element may have a design that allows the bone anchoring element with a pressure element adapted thereto to be pivoted only in a single plane. For example, the head may have at least one flat surface portion extending substantially parallel to the shank axis and the pressure element may have a cooperating portion to limit the pivoting to a single plane. Any design for providing an enlarged pivot angle may be used also. For the locking element all kinds of locking devices can be used, such as bayonet-type locking devices, two-part locking devices that allow to clamp the rod and the head independently with two locking elements, outer locking nuts, etc..

For the bone anchoring element all kinds of bone anchors can be used, such as screws, nails with or without barbs, cannulated bone anchors, two-part bone anchors, where head and shank are separate parts that can be assembled and other bone anchoring elements.

## Claims

1. Instrument for use with a polyaxial bone anchoring device, wherein the polyaxial bone anchoring device includes a receiving part (5) for receiving a rod (100) and for accommodating a head (3) of a bone anchoring element (1), wherein the head (3) can be clamped by exerting pressure onto it via a clamping element (7) and wherein the pressure can be adjusted using the instrument, the instrument including
a first member (10) including a first tubular portion (11) comprising a front end (11a), the first tubular portion (11) having near the front end (11a) a first engagement portion (12a, 12b) that is configured to engage the receiving part (5) in a positive-fit manner;
a second member (20, 21) having a front end (20a) and a second engagement portion (22, 22a) that is configured to engage the clamping element (7) and apply torque to the clamping element;
wherein in a first configuration the second member (20, 21) is only axially displaceable along a longitudinal axis (C) relative to the first member (10, 11) and wherein
in a second configuration the second member (20, 21) is rotatable with respect to the first member (10).

2. The instrument of claim 1, wherein in the second configuration the second member (20, 21) is rotatable with respect to the first member (10, 11) in a limited angular range around the longitudinal axis (C).

3. The instrument of claim 2, wherein the limited angular range is less than about 180°.

4. The instrument of one of claims 1 to 3, wherein the first member (10, 11) and the second member (20, 21) include cooperating third and fourth engagement portions (30, 24), that permit a linear displacement relative to each other while preventing a rotational movement of the second member (20, 21) relative to the first member (10, 11) and wherein the third engagement portion (30) is provided at the first member (10, 11) and the second engagement portion is provided at the second member (20, 21) or vice versa.

5. The instrument of claim 4, wherein the third engagement portion includes a longitudinal groove (24) and the fourth engagement portion includes a protrusion (30) engaging the longitudinal groove (24).

6. The instrument of one of claims 4 or 5, wherein a fifth engagement portion (25) is provided that is configured to cooperate with the fourth engagement portion (30) to permit a rotational movement between the first member (10, 11) and the second member (20, 21) in the second configuration.

7. The instrument of claim 6, therein the fifth engagement portion (25) is a horizontal recess that extends substantially perpendicular to the longitudinal axis (C) and wherein preferably the fifth engagement portion is in connection with the third engagement portion (24).

8. The instrument of one of claims 1 to 7, wherein the first member (10, 11) comprises a stop (14) for limiting an axial movement of the first member relative to the receiving part (5).

9. The instrument of one of claims 4 to 8, wherein the third and fourth engagement portions (24, 30) define a mounting position of the second member (20, 21) relative to the first member (10, 11) in an circumferential direction around the longitudinal axis (C) such that mounting is possible only when the second member (20, 21) is arranged relative to the first member (10, 11) when the third engagement portion (30) engages the fourth engagement portion (24).

10. The instrument of one of claims 1 to 9, wherein the second member (20, 21) is insertable into the first tubular portion (11) of the first member (10), and wherein preferably the second member includes a second tubular portion (21) with a front end (20a) that comprises the second engagement portions (22, 22a).

11. A system of an instrument and a polyaxial bone anchoring device, wherein the polyaxial bone anchoring device includes a receiving part (5) for receiving a rod (100) and for accommodating a head (3) of a bone anchoring element and a clamping element (7), wherein the head (3) can be clamped by exerting pressure onto it via the clamping element (7) and wherein the pressure can be adjusted using the instrument, wherein the instrument is an instrument according to one of claims 1 to 10.

12. A system of an instrument and a polyaxial bone anchoring device,
the polyaxial bone anchoring device including a receiving part (5) for receiving a rod (100) and for accommodating a head (3) of a bone anchoring element, wherein the head (3) can be clamped by exerting pressure onto it via a clamping element (7) and wherein the pressure can be adjusted using the instrument,
wherein the receiving part (5) has a first end (5a) and a second end (5b), a central axis (C) extending through the first end (5a) and the second end (5b) and coinciding with a longitudinal axis of the instrument, a channel (52) for receiving a rod, wherein the receiving part (5) further defines an accommodation space (55) for accommodating a head (3) of a bone anchoring element (1), the accommodation space having an opening (56) for inserting the head; and
wherein a pressure element (6) is provided that is arranged at least partially in the accommodation space (55), the pressure element (6) comprising a flexible portion (63) to clamp an inserted head,
wherein a clamping element (7) is provided, the clamping element (7) extending at least partially around the flexible portion (63), wherein the clamping element (7) is configured to move from a first position to a second position in which it exerts a clamping force onto the pressure element (6) and wherein the movement includes rotating the clamping element around the central axis (C) using the instrument,
the instrument including
a first member (10, 11) having a first engagement portion (12a, 12b) that is configured to engage the receiving part (5) in a positive-fit manner;
a second member (20, 21) having a front end (20a) and a second engagement portion (22, 22a) that is configured to engage the clamping element (7) and apply torque to the clamping element;
wherein in a first configuration the second member (20, 21) is only axially displaceable along a longitudinal axis (C) relative to the first member (10, 11) and wherein
in a second configuration the second member (20, 21) is rotatable with respect to the first member (10, 11).

13. The system of claim 11 or 12, wherein the clamping element (7) comprises an engagement portion (75) for engagement with the second engagement portion (22, 22a) of the second member (20, 21) of the instrument.

14. The system of one of claims 11 to 13, wherein the receiving part (5) comprises an outer surface portion (58a, 58b) that cooperates with the first engagement portion (12a, 12b) of the first member (10, 11) of the instrument to prevent rotation of the first member (10, 11) relative to the receiving part.

15. The system of one of claims 11 to 14, wherein the receiving part (5) and the clamping element (7) comprise an advancement structure (73, 9a, 9b) that is configured to permit an axial advancement of the clamping element (7) when the clamping element is rotated about the central axis (C) using the instrument wherein the second engagement portion (22, 22a) of the second member (20, 21) engages the clamping element (7) and the instrument is in the second configuration.

## Patentansprüche

1. Instrument zum Verwenden mit einer polyaxialen Knochenverankerungsvorrichtung, wobei die polyaxiale Knochenverankerungsvorrichtung ein Aufnahmeteil (5) zum Aufnehmen eines Stabs (100) und zum Aufnehmen eines Kopfs (3) eines Knochenverankerungselements (1) umfasst, wobei der Kopf (3) durch Ausüben von Druck auf ihn via einem Klemmelement (7) geklemmt werden kann und wobei der Druck durch Verwenden des Instruments eingestellt werden kann, wobei das Instrument umfasst
ein erstes Element (10) umfassend einen rohrförmigen Abschnitt (11), welcher ein vorderes Ende (11a) umfasst, wobei der erste rohrförmige Abschnitt (11) nahe dem vorderen Ende (11a) einen ersten Eingreifabschnitt (12a, 12b) aufweist, welcher ausgebildet ist, um in einer formschlüssigen Weise in das Aufnahmeteil (5) einzugreifen;
ein zweites Element (20, 21), welches ein vorderes Ende (20a) und einen zweiten Eingreifabschnitt (22, 22a) aufweist, der ausgebildet ist, um in das Klemmelement (7) einzugreifen und ein Drehmoment auf das Klemmelement zu applizieren;
wobei das zweite Element (20, 21) in einer ersten Konfiguration lediglich axial entlang einer Längsachse (C) relativ zu dem ersten Element (10, 11) verschiebbar ist und wobei
das zweite Element (20, 21) in einer zweiten Konfiguration relativ zu dem ersten Element (10) rotierbar ist.

2. Instrument gemäß Anspruch 1, wobei, das zweite Element (20, 21) in der zweiten Konfiguration relativ zu dem ersten Element (10, 11) in einem limitierten Winkelbereich um die Längsachse (C) herum rotierbar ist.

3. Instrument gemäß Anspruch 2, wobei der limitierte Winkelbereich kleiner ist als ungefähr 180°.

4. Instrument gemäß einem der Ansprüche 1 bis 3, wobei das erste Element (10, 11) und das zweite Element (20, 21) zusammenwirkende dritte und vierte Eingreifabschnitte (30, 24) umfassen, welche eine lineare Verschiebung relativ zueinander erlauben, während sie eine Rotationsbewegung des zweiten Elements (20, 21) relativ zu dem ersten Element (10, 11) verhindern, und wobei der dritte Eingreifabschnitt (30) an dem ersten Element (10, 11) vorgesehen ist und der zweite Eingreifabschnitt an dem zweiten Element (20, 21) vorgesehen ist oder umgekehrt.

5. Instrument gemäß Anspruch 4, wobei der dritte Eingreifabschnitt eine längliche Nut (24) umfasst und der vierte Eingreifabschnitt einen Vorsprung (30) umfasst, welcher in die längliche Nut (24) eingreift.

6. Instrument gemäß einem der Ansprüche 4 oder 5, wobei ein fünfter Eingreifabschnitt (25) vorgesehen ist, welcher ausgebildet ist, um mit dem vierten Eingreifabschnitt (30) zusammenzuwirken, um in der zweiten Konfiguration eine Rotationsbewegung zwischen dem ersten Element (10, 11) und dem zweiten Element (20, 21) zu erlauben.

7. Instrument gemäß Anspruch 6, wobei der fünfte Eingreifabschnitt (25) eine horizontale Ausnehmung ist, welche sich im Wesentlichen senkrecht zu der Längsachse (C) erstreckt und wobei vorzugsweise der fünfte Eingreifabschnitt mit dem dritten Eingreifabschnitt (24) in Verbindung ist.

8. Instrument gemäß einem der Ansprüche 1 bis 7, wobei das erste Element (10, 11) einen Anschlag (14) zum Limitieren einer axialen Bewegung des ersten Elements relativ zu dem Aufnahmeteil (5) aufweist.

9. Instrument gemäß einem der Ansprüche 4 bis 8, wobei der dritte und der vierte Eingreifabschnitt (24, 30) eine Einbauposition des zweiten Elements (20, 21) relativ zu dem ersten Element (10, 11) in einer um die Längsachse (C) herum umlaufenden Richtung derart definieren, dass ein Einbauen lediglich möglich ist, wenn das zweite Element (20, 21) relativ zu dem ersten Element (10, 11) angeordnet ist, wenn der dritte Eingreifabschnitt (30) in den vierten Eingreifabschnitt (24) eingreift.

10. Instrument gemäß einem der Ansprüche 1 bis 9, wobei das zweite Element (20, 21) in den ersten rohrförmigen Abschnitt (11) des ersten Elements (10) einführbar ist, und wobei vorzugsweise das zweite Element einen zweiten rohrförmigen Abschnitt (21) mit einem vorderen Ende (20a) umfasst, welcher die zweiten Eingreifabschnitte (22, 22a) umfasst.

11. System aus einem Instrument und einer polyaxialen Knochenverankerungsvorrichtung, wobei die polyaxiale Knochenverankerungsvorrichtung ein Aufnahmeteil (5) zum Aufnehmen eines Stabs (100) und zum Aufnehmen eines Kopfs (3) eines Knochenverankerungselements umfasst und ein Klemmelement (7),
wobei der Kopf (3) durch Ausüben von Druck auf ihn via dem Klemmelement (7) geklemmt werden kann und wobei der Druck durch Verwenden des Instruments eingestellt werden kann, wobei das Instrument ein Instrument gemäß einem der Ansprüche 1 bis 10 ist.

12. System aus einem Instrument und einer polyaxialen Knochenverankerungsvorrichtung, wobei die polyaxiale Knochenverankerungsvorrichtung ein Aufnahmeteil (5) zum Aufnehmen eines Stabs (100) und zum Aufnehmen eines Kopfs (3) eines Knochenverankerungselements umfasst, wobei der Kopf (3) durch Ausüben von Druck auf ihn via dem Klemmelement (7) geklemmt werden kann, und wobei der Druck durch Verwenden des Instruments eingestellt werden kann,
wobei das Aufnahmeteil (5) ein erstes Ende (5a) und ein zweites Ende (5b), eine Zentralachse (C), welche sich durch das erste Ende (5a) und das zweite Ende (5b) erstreckt und mit einer Längsachse (C) des Instruments zusammenfällt, und einen Kanal (52) zum Aufnehmen eines Stabs aufweist, wobei das Aufnahmeteil (5) des Weiteren einen Aufnahmeraum (55) zum Aufnehmen eines Kopfs (3) eines Knochenverankerungselements (1) definiert, wobei der Aufnahmeraum eine Öffnung (56) zum Einführen des Kopfs aufweist; und
wobei ein Druckelement (6) vorgesehen ist, welches zumindest teilweise in dem Aufnahmeraum (55) angeordnet ist, wobei das Druckelement (6) einen flexiblen Abschnitt (63) zum Klemmen des eingeführten Kopfs (3) aufweist,
wobei ein Klemmelement (7) vorgesehen ist, wobei sich das Klemmelement (7) zumindest teilweise um den flexiblen Abschnitt (63) herum erstreckt, wobei das Klemmelement (7) ausgebildet ist, um sich von einer ersten Position zu einer zweiten Position zu bewegen, in welcher es eine Klemmkraft auf das Druckelement (6) ausübt und wobei die Bewegung Rotieren des Klemmelements um die Zentralachse (C) durch Verwenden des Instruments umfasst,
wobei das Instrument umfasst
ein erstes Element (10, 11), welches einen ersten Eingreifabschnitt (12a, 12b) aufweist, der ausgebildet ist, um in einer formschlüssigen Weise in das Aufnahmeteil (5) einzugreifen;
ein zweites Element (20, 21), welches ein vorderes Ende (20a) und einen zweiten Eingreifabschnitt (22, 22a) aufweist, der ausgebildet ist, um in das Klemmelement (7) einzugreifen und ein Drehmoment auf das Klemmelement zu applizieren;
wobei das zweite Element (20, 21) in einer ersten Konfiguration lediglich axial entlang einer Längsachse (C) relativ zu dem ersten Element (10, 11) verschiebbar ist, und wobei das zweite Element (20, 21) in einer zweiten Konfiguration relativ zu dem ersten Element (10, 11) rotierbar ist.

13. System gemäß Anspruch 11 oder 12, wobei das Klemmelement (7) einen Eingreifabschnitt (75) zum Eingreifen in einen zweiten Eingreifabschnitt (22, 22a) des zweiten Elements (20, 21) des Instruments umfasst.

14. System gemäß einem der Ansprüche 11 bis 13, wobei das Aufnahmeteil (5) einen Außenflächenabschnitt (58a, 58b) umfasst, welcher mit dem ersten Eingreifabschnitt (12a, 12b) des ersten Elements (10, 11) des Instruments zusammenwirkt, um eine Rotation des ersten Elements (10, 11) relativ zu dem Aufnahmeteil zu verhindern.

15. System gemäß einem der Ansprüche 11 bis 14, wobei das Aufnahmeteil (5) und das Klemmelement (7) eine Vorrückstruktur (73, 9a, 9b) umfassen, welche eingerichtet ist, ein axiales Verrücken des Klemmelements (7) zu erlaubten, wenn das Klemmelement durch Verwenden des Instruments um die Ventralachse (C) rotiert wird, wobei der zweite Eingreifabschnitt (22, 22a) des zweiten Elements (20, 21) in das Klemmelement (7) eingreift und das Instrument in der zweiten Konfiguration ist.

## Revendications

1. Instrument destiné à être utilisé avec un dispositif d'ancrage osseux polyaxial, dans lequel le dispositif d'ancrage osseux polyaxial comprend une partie de réception (5) destinée à recevoir une tige (100) et à constituer un logement pour une tête (3) d'un élément d'ancrage osseux (1), dans lequel la tête (3) peut être fixée par serrage par l'exercice d'une pression sur celle-ci par le biais d'un élément de fixation par serrage (7), et dans lequel la pression peut être ajustée au moyen de l'instrument, l'instrument comprenant
un premier organe (10) comprenant une première partie tubulaire (11), laquelle comporte une extrémité avant (11a), la première partie tubulaire (11) comportant, à proximité de l'extrémité avant (11a), une première partie d'accouplement (12a, 12b) qui est configurée pour s'accoupler avec la partie de réception (5) par complémentarité de forme;
un second organe (20, 21) comportant une extrémité avant (20a) et une deuxième partie d'accouplement (22, 22a) qui est configurée pour s'accoupler avec l'élément de fixation par serrage (7) et appliquer un couple à l'élément de fixation par serrage ;
dans lequel, dans une première configuration, le second organe (20, 21) ne peut être déplacé qu'axialement le long d'un axe longitudinal (C) par rapport au premier organe (10, 11), et dans lequel
dans une seconde configuration, le second organe (20, 21) peut être mis en rotation par rapport au premier organe (10).

2. Instrument selon la revendication 1, dans lequel, dans la seconde configuration, le second organe (20, 21) peut être mis en rotation par rapport au premier organe (10, 11) au sein d'une plage angulaire limitée autour de l'axe longitudinal (C).

3. Instrument selon la revendication 2, dans lequel la plage angulaire limitée est inférieure à environ 180°.

4. Instrument selon l'une des revendications 1 à 3, dans lequel le premier organe (10, 11) et le second organe (20, 21) comprennent des troisième et quatrième parties d'accouplement (30, 24) coopérantes, qui permettent un déplacement linéaire de l'une par rapport à l'autre tout en empêchant un mouvement de rotation du second organe (20, 21) par rapport au premier organe (10, 11), et dans lequel la troisième partie d'accouplement (30) est placée au niveau du premier organe (10, 11) et la deuxième partie d'accouplement est placée au niveau du second organe (20, 21) ou vice versa.

5. Instrument selon la revendication 4, dans lequel la troisième partie d'accouplement comprend une rainure longitudinale (24) et la quatrième partie d'accouplement comprend une saillie (30) s'accouplant avec la rainure longitudinale (24).

6. Instrument selon l'une des revendications 4 ou 5, dans lequel une cinquième partie d'accouplement (25) est prévue, celle-ci étant configurée pour coopérer avec la quatrième partie d'accouplement (30) de façon à permettre un mouvement de rotation entre le premier organe (10, 11) et le second organe (20, 21) dans la seconde configuration.

7. Instrument selon la revendication 6, dans lequel la cinquième partie d'accouplement (25) est un évidement horizontal qui s'étend de manière substantiellement perpendiculaire à l'axe longitudinal (C), et dans lequel, de préférence, la cinquième partie d'accouplement est reliée à la troisième partie d'accouplement (24).

8. Instrument selon l'une des revendications 1 à 7, dans lequel le premier organe (10, 11) comprend une butée (14) destinée à limiter un mouvement axial du premier organe par rapport à la partie de réception (5).

9. Instrument selon l'une des revendications 4 à 8, dans lequel les troisième et quatrième parties d'accouplement (24, 30) définissent une position d'assemblage du second organe (20, 21) par rapport au premier organe (10, 11) dans une direction circonférentielle autour de l'axe longitudinal (C), de telle sorte que l'assemblage soit possible uniquement lorsque le second organe (20, 21) est disposé par rapport au premier organe (10, 11) lorsque la troisième partie d'accouplement (30) s'accouple avec la quatrième partie d'accouplement (24).

10. Instrument selon l'une des revendications 1 à 9, dans lequel le second organe (20, 21) peut être inséré dans la première partie tubulaire (11) du premier organe (10), et dans lequel, de préférence, le second organe comprend une seconde partie tubulaire (21) comportant une extrémité avant (20a) qui comprend les deuxièmes parties d'accouplement (22, 22a).

11. Système constitué d'un instrument et d'un dispositif d'ancrage osseux polyaxial, dans lequel le dispositif d'ancrage osseux polyaxial comprend une partie de réception (5) destinée à recevoir une tige (100) et à constituer un logement pour une tête (3) d'un élément d'ancrage osseux et un élément de fixation par serrage (7), dans lequel la tête (3) peut être fixée par serrage par l'exercice d'une pression sur celle-ci par le biais de l'élément de fixation par serrage (7), et dans lequel la pression peut être ajustée au moyen de l'instrument, l'instrument étant un instrument selon l'une des revendications 1 à 10.

12. Système constitué d'un instrument et d'un dispositif d'ancrage osseux polyaxial,
le dispositif d'ancrage osseux polyaxial comprenant une partie de réception (5) destinée à recevoir une tige (100) et à constituer un logement pour une tête (3) d'un élément d'ancrage osseux, dans lequel la tête (3) peut être fixée par serrage par l'exercice d'une pression sur celle-ci par le biais d'un élément de fixation par serrage (7), et dans lequel la pression peut être ajustée au moyen de l'instrument,
dans lequel la partie de réception (5) comporte une première extrémité (5a) et une seconde extrémité (5b), un axe central (C) s'étendant à travers la première extrémité (5a) et la seconde extrémité (5b) et coïncidant avec un axe longitudinal de l'instrument, un canal (52) destiné à recevoir une tige, dans lequel la partie de réception (5) définit en outre un espace formant logement (55) destiné à constituer un logement pour une tête (3) d'un élément d'ancrage osseux (1), l'espace formant logement comportant une couverture (56) pour l'insertion de la tête ; et
dans lequel un élément de pression (6) est prévu, celui-ci étant disposé au moins partiellement dans l'espace formant logement (55), l'élément de pression (6) comprenant une partie souple (63) destinée à fixer par serrage une tête insérée,
dans lequel un élément de fixation par serrage (7) est prévu, l'élément de fixation par serrage (7) s'étendant au moins partiellement autour de la partie souple (63), dans lequel l'élément de fixation par serrage (7) est configuré pour se déplacer d'une première position à une seconde position, dans laquelle il exerce une force de fixation par serrage sur l'élément de pression (6), et dans lequel le déplacement comprend une rotation de l'élément de fixation par serrage autour de l'axe central (C) au moyen de l'instrument,
l'instrument comprenant
un premier organe (10, 11) comportant une première partie d'accouplement (12a, 12b) qui est configurée pour s'accoupler avec la partie de réception (5) par complémentarité de forme ;
un second organe (20, 21) comportant une extrémité avant (20a) et une deuxième partie d'accouplement (22, 22a) qui est configurée pour s'accoupler avec l'élément de fixation par serrage (7) et appliquer un couple à l'élément de fixation par serrage ;
dans lequel, dans une première configuration, le second organe (20, 21) ne peut être déplacé qu'axialement le long d'un axe longitudinal (C) par rapport au premier organe (10, 11), et dans lequel,
dans une seconde configuration, le second organe (20, 21) peut être mis en rotation par rapport au premier organe (10, 11).

13. Système selon la revendication 11 ou 12, dans lequel l'élément de fixation par serrage (7) comprend une partie d'accouplement (75) destinée à s'accoupler avec la deuxième partie d'accouplement (22, 22a) du second organe (20, 21) de l'instrument.

14. Système selon l'une des revendications 11 à 13, dans lequel la partie de réception (5) comprend une partie de surface extérieure (58a, 58b) qui coopère avec la première partie d'accouplement (12a, 12b) du premier organe (10, 11) de l'instrument, de façon à empêcher une rotation du premier organe (10, 11) par rapport à la partie de réception.

15. Système selon l'une des revendications 11 à 14, dans lequel la partie de réception (5) et l'élément de fixation par serrage (7) comprennent une structure d'avancée (73, 9a, 9b) qui est configurée pour permettre une avancée axiale de l'élément de fixation par serrage (7) lorsque l'élément de fixation par serrage est mis en rotation autour de l'axe central (C) au moyen de l'instrument, dans lequel la deuxième partie d'accouplement (22, 22a) du second organe (20, 21) s'accouple avec l'élément de fixation par serrage (7) et l'instrument se trouve dans la seconde configuration.
